# FASCICULE DE BREVET EUROPEEN

(11) **EP 3 440 202 B1**
(45) Date de publication et mention de la délivrance du brevet: **16.11.2022**
(21) Numéro de dépôt: 17712173.8
(22) Date de dépôt: 24.03.2017
(51) Int. Cl.: C12N 9/42, C12P 7/10

(54) **PROCEDE DE PRODUCTION DE CELLULASES AVEC DU MARC LIGNOCELLULOSIQUE PRETRAITE**
VERFAHREN ZUR HERSTELLUNG VON CELLULASEN MIT VORBEHANDELTEM LIGNOCELLULOSISCHEM TRESTER
METHOD FOR PRODUCING CELLULASES WITH PRETREATED LIGNOCELLULOSIC POMACE

(30) Priorité: 08.04.2016 FR 1653124
(43) Date de publication de la demande: 13.02.2019
(73) Titulaire: IFP Energies nouvelles, 92852 Rueil-Malmaison Cedex (FR); Agro Industries Recherche Et Developpement, 51110 Pomacle (FR); Institut national de recherche pour l'agriculture, l'alimentation et l'environnement, 75007 Paris (FR)
(72) Inventeur: BEN CHAABANE, Fadhel, 75020 Paris (FR); COHEN, Celine, 75017 Paris (FR)
(86) Numéro de dépôt international: PCT/EP2017/057121
(87) Numéro de publication internationale: WO 2017/174378

(56) Documents cités:
- WO-A1-2013/054005
- WO-A1-2013/087998
- WO-A1-2015/091079
- WO-A2-2007/005918
- VERA NOVY ET AL: "From wheat straw to bioethanol: integrative analysis of a separate hydrolysis and co-fermentation process with implemented enzyme production", BIOTECHNOLOGY FOR BIOFUELS, BIOMED CENTRAL LTD, GB, vol. 8, no. 1, 18 mars 2015 (2015-03-18), page 46, XP021215584, ISSN: 1754-6834, DOI: 10.1186/S13068-015-0232-0
- KIM TAE HYUN ET AL: "Bioconversion of sawdust into ethanol using dilute sulfuric acid-assisted continuous twin screw-driven reactor pretreatment and fed-batch simultaneous saccharification and fermentation", BIORESOURCE TECHNOLOGY, ELSEVIER BV, GB, vol. 130, 8 décembre 2012 (2012-12-08), pages 306-313, XP028980583, ISSN: 0960-8524, DOI: 10.1016/J.BIORTECH.2012.11.125
- ROSGAARD L ET AL: "Effects of substrate loading on enzymatic hydrolysis and viscosity of pretreated barley straw", APPLIED BIOCHEMISTRY AND BIOTECHNOLOGY, HUMANA PRESS, INC, UNITED STATES, vol. 143, no. 1, octobre 2007 (2007-10), pages 27-40, XP002668850, ISSN: 0273-2289, DOI: 10.1007/S12010-007-0028-1 [extrait le 2007-04-17]
- MONSCHEIN MAREIKE ET AL: "Effect of pretreatment severity in continuous steam explosion on enzymatic conversion of wheat straw: Evidence from kinetic analysis of hydrolysis time courses", BIORESOURCE TECHNOLOGY, ELSEVIER BV, GB, vol. 200, 20 octobre 2015 (2015-10-20), pages 287-296, XP029312773, ISSN: 0960-8524, DOI: 10.1016/J.BIORTECH.2015.10.020

## Description

L'invention concerne un procédé de production d'enzymes cellulolytiques et hémicellulolytiques.

Les enzymes sont utilisées notamment dans les procédés de production de biocarburants de deuxième génération (i.e. à partir de biomasse ligno-cellulosique), et en particulier lorsque le biocarburant est l'éthanol. De façon générale, le procédé selon l'invention est applicable dans tous les procédés incluant une hydrolyse enzymatique de biomasse, et notamment de biomasse lignocellulosique. Il est en particulier utilisable dans les productions d'enzymes par des champignons filamenteux.

### Art antérieur

Depuis plus de 45 ans, la transformation de matériaux ligno-cellulosique en éthanol, après hydrolyse des polysaccharides constitutifs en sucres fermentescibles, a fait l'objet de très nombreux travaux.

Les matériaux ligno-cellulosiques sont des matériaux cellulosiques, c'est-à-dire constitués à plus de 90% poids de cellulose et/ou lignocellulose (la lignocellulose comprend essentiellement de la cellulose, de l'hémicellulose et de la lignine). Les celluloses et hémicelluloses sont des polysaccharides essentiellement constitués de pentoses et d'hexoses. La lignine est une macromolécule de structure complexe et de haut poids moléculaire, à base de composés phénoliques.

Le bois, les pailles, les rafles de maïs sont les matériaux ligno-cellulosiques les plus utilisés, mais d'autres ressources telles que des cultures forestières dédiées, résidus de plantes alcooligènes, sucrières et céréalières, résidus lignocellulosiques de l'industrie papetière et des produits de la transformation des matériaux ligno-cellulosiques sont utilisables. Ils comprennent pour la plupart environ 35 à 50 % de cellulose, 20 à 30 % d'hémicellulose et 15 à 25 % de lignine.

Le procédé de transformation biochimique des matériaux ligno-cellulosiques en éthanol comprend une étape de prétraitement physico-chimique, suivie d'une étape d'hydrolyse enzymatique utilisant un cocktail enzymatique pour produire des sucres, d'une étape de fermentation éthanolique des sucres libérés, la fermentation éthanolique et l'hydrolyse enzymatique pouvant être conduites simultanément (procédé SSF), et d'une étape de purification de l'éthanol.

Le cocktail enzymatique est un mélange d'enzymes cellulolytiques (également appelées cellulases) et/ou hémicellulolytiques (appelées souvent xylanases). Les enzymes cellulolytiques présentent trois grands types d'activités : endoglucanases, exoglucanases et cellobiases, ces dernières étant également appelées β glucosidases. Les enzymes hémicellulolytiques présentent notamment des activités xylanases.

L'hydrolyse enzymatique est efficace et s'effectue dans des conditions douces.

Le coût des enzymes reste très élevé, il représente de 20 à 50% du coût de transformation du matériau ligno-cellulosique en éthanol. De ce fait, beaucoup de travaux ont été conduits pour réduire ce coût : tout d'abord sur l'optimisation de la production d'enzymes, en sélectionnant les microorganismes hyperproducteurs et en améliorant les procédés de production desdites enzymes, puis sur la diminution de la quantité d'enzymes en hydrolyse, en optimisant l'étape de prétraitement, en améliorant l'activité spécifique de ces enzymes, et en optimisant la mise en œuvre de l'étape d'hydrolyse enzymatique.

Au cours de la dernière décennie, de nombreux travaux se sont attachés à comprendre les mécanismes d'action et d'expression du cocktail enzymatique. Le but est de faire sécréter le cocktail le plus approprié à l'hydrolyse des matériaux ligno-cellulosiques en modifiant les microorganismes.

Le microorganisme cellulolytique le plus utilisé pour la production industrielle du cocktail enzymatique est le champignon *Trichoderma reesei.* Il a la faculté de sécréter, en présence d'un substrat carboné inducteur, la cellulose par exemple, un cocktail enzymatique à des concentrations très élevées (pouvant aller jusqu'à 100 g/L). D'autres protéines possédant des propriétés indispensables à l'hydrolyse des matériaux ligno-cellulosiques sont également produites par *Trichoderma reesei,* les xylanases par exemple. La présence d'un substrat carboné inducteur est indispensable à l'expression des enzymes cellulolytiques et/ou hémicellulolytiques. La nature du substrat carboné a une forte influence sur la composition du cocktail enzymatique. C'est le cas du xylose, qui, associé à un substrat carboné inducteur comme la cellulose ou le lactose, permet d'améliorer significativement l'activité dite xylanase.

Le lactose reste, dans un procédé de production industriel de cocktail enzymatique, un des substrats les plus appropriés; son coût varie cependant de manière importante et représente environ de un à deux tiers du prix de revient des enzymes. Dans le cas de l'utilisation de lactose comme substrat carboné, le procédé de production de cocktail enzymatique est dépendant d'une source de carbone extérieure. De ce fait, l'utilisation de substrats carbonés issus du procédé de conversion biochimique de matériaux ligno-cellulosiques est une voie de progrès importante.

Un autre substrat inducteur qui peut être utilisé est la cellulose. Cependant celle-ci a un coût encore plus élevé que le lactose.

La demande de brevet US-2011/262997 remplace la cellulose utilisée dans des procédés classiques de production de cellulases par de la biomasse prétraitée, en particulier par explosion à la vapeur en condition acide, biomasse qui a été éventuellement lavée. La biomasse prétraitée est utilisée seulement comme inducteur, la croissance du microorganisme étant obtenue avec du glucose comme substrat carboné. Dans les exemples du procédé opérés en batch, le marc prétraité (par cuisson acide) et lavé est ajouté au début de l'expérience en totalité ainsi qu'une solution de glucose et d'antimousse.

D'une part, ce procédé a pour inconvénient de nécessiter la détoxification du marc prétraité utilisé avant son utilisation. Des lavages sont une option proposée pour cela. Si ce procédé devait être appliqué à l'échelle industrielle, la mise en œuvre de lavages augmenterait fortement le coût du procédé.

D'autre part, le fait de rajouter tout le marc prétraité dès le début de l'expérience augmente fortement la viscosité du milieu, ce qui nécessite d'appliquer des puissances dissipées élevées. On appelle « puissance dissipée » la puissance du moteur (kW/m3) nécessaire pour l'agitation du milieu. Cette augmentation de viscosité nécessite également des débits d'aération très élevés pour permettre un transfert d'oxygène suffisant.

Une autre demande de brevet WO-13/190064 concerne également la production d' enzymes pour l'hydrolyse enzymatique de biomasse lignocellulosique prétraitée (dit 1^{ere} biomasse prétraitée). Cette production est effectuée en l'absence d'ajout de sucre (comme le glucose), mais en présence d'un solide comprenant des sucres complexes et de la lignine. Ce solide est de préférence issu d'un autre procédé de traitement de biomasse qui comprend l'hydrolyse enzymatique puis la séparation dudit solide, procédé opérant à partir d'une 2^{e} biomasse prétraitée. Dans ce procédé de production d'enzymes, il est essentiel que le ratio sucres complexes / lignine dudit solide soit inférieur au ratio sucres complexes / lignine de ladite 2^{e} biomasse. Ce critère nécessite de retirer au moins 50% de l'eau et des sucres solubles de l'hydrolysat. Ceci augmente le coût du procédé. Par ailleurs, ce solide comporte un pourcentage très élevé de lignine, ce qui rend la production d'enzymes moins efficace. En effet, il est connu que les composés phénolés de la lignine ont un effet inhibiteur sur les enzymes.

La demande de brevet WO-13/053924 opère avec un procédé du même type, la biomasse prétraitée joue également le rôle de substrat de croissance, sans ajout (ou avec ajout faible) de sucre simple (glucose). Par ailleurs, la culture se fait en l'absence d'ajout de vitamines et/ou minéraux et/ou inducteurs de production d'enzymes. Ce procédé nécessite recommande également la détoxification du marc (notamment si le prétraitement utilisé est acide).

La demande de brevet WO11028554 enseigne l'utilisation du résidu solide issu de l'hydrolyse des hémicelluloses pour la production de cellulases par *Trichoderma reesei,* le résidu ayant été débarrassé de sa fraction lignine dans une étape d'extraction de la lignine. La production est effectuée en présence d'un apport de sucres (glucose). Les enzymes obtenues sont utilisées pour l'hydrolyse de la cellulose, et non pas pour l'hydrolyse de l'hémicellulose. Le résidu solide délignifié est utilisé dès le début de la phase de croissance du microorganisme, ce qui induit des difficultés d'opération.

Un objet de l'invention est de proposer une source de carbone inductrice issue du procédé de production et permettant de produire un cocktail enzymatique approprié à l'hydrolyse du matériau ligno-cellulosique.

Le procédé de production de cellulases utilise un marc prétraité de préférence non préalablement détoxifié.

### Description détaillée de l'invention

La présente invention concerne un procédé de production d'enzymes cellulolytiques ou hémicellulolytiques en culture submergée par un microorganisme cellulolytique, ledit microorganisme cellulolytique étant un champignon de l'espèce Trichoderma *reesei*, qui utilise un marc prétraité tel que défini à la revendication 1.

L'invention est exposée dans le jeu de revendications joint.

On appelle « marc prétraité » le substrat issu de matériaux lignocellulosique ayant subi une étape de prétraitement, de préférence une explosion à la vapeur en milieu acide. La biomasse est un type de matériau préféré ; dans le texte les termes biomasse et matériau lignocellulosique sont souvent utilisés de façon égale.

Un procédé de conversion biochimique de matériaux ligno-cellulosiques en alcool (en particulier en éthanol) comprend généralement une étape de prétraitement physico-chimique (de préférence une explosion à la vapeur en milieu acide) produisant un marc prétraité, suivie d'une étape d'hydrolyse enzymatique utilisant un cocktail enzymatique produisant des sucres, puis une étape de fermentation éthanolique desdits sucres, la fermentation éthanolique et l'hydrolyse enzymatique pouvant être conduites simultanément (procédé SSF) ou séparément (procédé SHF), et une étape de purification de l'éthanol.

L'invention présente de nombreux avantages :
- réduire, voire supprimer l'apport en substrat carboné d'origine externe audit procédé de conversion biochimique de matériaux ligno-cellulosiques.
- de produire un cocktail enzymatique particulièrement adapté à l'hydrolyse enzymatique du matériau lignocellulosique prétraité dans le procédé de conversion biochimique.
- proposer un mode de conduite de procédé qui permet de ne pas avoir à détoxifier (par ex laver) le marc prétraité utilisé. Cela permet de réduire la quantité d'effluents produits lors des lavages et qui doivent être retraités avant rejet.
- proposer un procédé où la viscosité du milieu est maintenue à une valeur basse, ce qui permet de limiter la demande en oxygène et avoir ainsi un procédé qui peut être extrapolé à l'échelle industrielle.

Le procédé peut être opéré en mode continu ou fed-batch.

En fed-batch, un mode particulièrement avantageux de conduite du procédé est de procéder à l'ajout du marc de façon séquentielle en fonction de l'évolution du pH du milieu réactionnel et du % de CO2 dans les gaz en sortie.

Ce mode de conduite permet d'utiliser du marc prétraité non détoxifié (non lavé).

Un avantage de ce procédé, et notamment avec le mode de conduite ci-dessus, est de maintenir une faible viscosité du milieu. Cela est important pour ne pas pénaliser le transfert d'oxygène et avoir un procédé extrapolable à l'échelle industrielle.

L'invention concerne plus particulièrement un procédé de production d'enzymes comprenant deux phases :
- une phase a) de croissance dudit champignon de l'espèce *Trichoderma reesei* en présence d'au moins un substrat carboné de croissance, en réacteur fermé, ladite phase de croissance étant réalisée avec une concentration en substrat carboné de croissance comprise entre 10 et 90 g/L. Le marc prétraité n'est pas introduit dans cette phase. On obtient ainsi une culture dudit microorganisme.
- une phase b) de production du cocktail enzymatique (ou enzymes) dans laquelle au moins un substrat carboné inducteur est ajouté en fed-batch ou en continu, ledit substrat carboné inducteur étant une partie dudit marc prétraité, ladite phase de production étant réalisée avec un ajout en continu ou non continu (fed-batch) du marc.

Plus précisément, l'invention concerne un procédé de production d'enzymes cellulolytiques ou hémicellulolytiques comprenant :
- une phase a) de croissance d'un microorganisme cellulolytique en réacteur fermé, en présence d'au moins un substrat carboné de croissance à une concentration comprise entre 10 et 90 g/L, à une température de 25- 30°C et un pH de 4 à 5,5,
- une phase b) de production des enzymes dans laquelle au moins un substrat carboné inducteur est introduit à une température de 25-27°C et un pH de 4-5,
procédé dans lequel
- ledit substrat inducteur est un marc prétraité issu d'un procédé de prétraitement de matériau lignocellulosique, ledit marc n'ayant pas subi d'hydrolyse enzymatique et étant introduit en mode fed-batch ou continu,
- ledit marc montre un rendement en hydrolyse enzymatique d'au moins 80% après 96h, ladite hydrolyse étant conduite à 50°C et pH 4.8 sur ledit marc à 15% pds de matière sèche (MS) avec 10 mg d'enzymes CELLIC^{®} CTEC2 par gramme de MS, ledit rendement étant le rapport de la masse de sucres simples libérés par l'hydrolyse enzymatique divisé par la masse théorique maximale qui serait obtenue si toute la cellulose, les hémicelluloses et les oligomères issus du prétraitement étaient hydrolysés,
- ledit marc prétraité mis en suspension à température ambiante, à 10% pds de MS, présente une viscosité apparente inférieure à 1 Pa.s pour un taux de cisaillement de 10 s-1, de préférence inférieure à 0.15 Pa.s.
- ledit marc est introduit à un débit compris entre 0.3 et 0.8 gramme de MS par litre de milieu et par heure en mode continu ; en mode fed-batch la quantité de marc ajoutée toutes les f heures, f étant compris entre 0,5h et 48h, est comprise entre 0.3f et 0.8f gramme de matière sèche par litre de milieu.

De préférence, la viscosité apparente du milieu de l'étape b) reste inférieure à 10 Pa.s pour un taux de cisaillement de 10 s-1, de préférence inférieure à 1 Pa.s.

Le marc peut avoir été détoxifié (lavé) avant d'être introduit dans la phase b) ou bien ne pas avoir été détoxifié (lavé). La phase b) opère généralement en l'absence de sucre ajouté. Ainsi, de façon très avantageuse, ledit marc prétraité est le seul substrat inducteur.

Comme cela sera détaillé plus loin, le marc prétraité est formé d'un liquide et d'un solide, le solide contient 20-70% de matière sèche dont 20-50% de lignine. Le solide dudit marc prétraité contient en outre 30-60% pds de cellulose et 1-10% pds de composés minéraux et d'hémicellulose, et le liquide contient 30-80% pds de sucres.

Le prétraitement est une explosion à la vapeur en condition acide.

### La phase a)

Les microorganismes mis en œuvre dans le procédé de production d'un cocktail enzymatique selon l'invention sont des souches de champignons appartenant à l'espèce *Trichoderma reesei.* Les souches industrielles les plus performantes sont les souches appartenant à l'espèce *Trichoderma reesei,* modifiées pour améliorer le cocktail enzymatique par les procédés de mutation-sélection, comme par exemple la souche CL847 (brevet FR-2555803). Les souches améliorées par les techniques de recombinaison génétique peuvent être également utilisées. Ces souches sont cultivées en réacteurs agités et aérés dans des conditions compatibles avec leur croissance et la production des enzymes. On connaît de nombreuses souches améliorées telles que MCG77 (Gallo - US Patent 4275 167), MCG 80 (Allen, AL and Andreotti, RE, Biotechnol-Bioengi 1982 12, 451-459 1982), RUT C30 (Montenecourt, BS and Eveleigh, DE, Appl. Environ. Microbiol. 1977, 34, 777-782) et CL847 (Durand et al, 1984 Proc. Symposium SFM "Genetics of Industrial Microorganisms ". Paris. HESLOT H. Ed, pp 39-50).

Le substrat carboné de croissance dudit microorganisme utilisé dans ladite phase a) est avantageusement choisi parmi les sucres solubles industriels, et de préférence parmi le glucose, le lactose, le xylose, les résidus liquides (vinasses) obtenus après fermentation éthanolique des sucres monomères des hydrolysats enzymatiques de matériaux lignocellulosique et les extraits de la fraction hémicellulosique (composés en C5) sous forme de monomères provenant de substrat lignocellulosique prétraité (tel que le liquide séparé au niveau du prétraitement), utilisé seul ou en mélange. Selon sa nature, ledit substrat carboné est introduit dans le réacteur fermé avant stérilisation ou est stérilisé séparément et introduit dans le réacteur fermé après stérilisation de ce dernier.

Ce substrat carboné de croissance est utilisé dans ladite phase a) à une concentration initiale comprise entre 10 et 90 g de substrat carboné par litre de volume réactionnel.

De préférence, ladite phase a) de croissance est réalisée sur une durée comprise entre 30 et 70 h, de préférence entre 30 et 40 h.

De préférence, ladite phase a) de croissance opère à un pH compris entre 4 et 5,5, et de préférence de 4,8 et à une température comprise entre 25 et 30°C, et de préférence de 27°C.

### La phase b)

Conformément à l'invention, ledit substrat carboné inducteur utilisé dans ladite phase b) de production est avantageusement un marc prétraité.

L'étape de prétraitement du matériau ligno-cellulosique permet d'améliorer la susceptibilité de la fraction cellulosique à l'hydrolyse enzymatique.

De préférence, l'étape de prétraitement est effectuée en milieu acide. C'est de préférence une explosion à la vapeur en condition acide. L'explosion à la vapeur est précédée d'une étape d'imprégnation dudit matériau lignocellulosique avec une solution acide, qui est de préférence une solution aqueuse d'acide sulfurique. On parle alors d'explosion à la vapeur en condition acide (le matériau contient de l'acide).

A l'issue du prétraitement, il est obtenu un marc prétraité et une partie de ce marc est prélevée pour la production d'enzymes, l'autre partie est envoyée en hydrolyse enzymatique puis en fermentation pour la production d'alcool.

Ladite partie du marc peut être utilisée telle que (marc entier) ou, de préférence, ce peut être sa partie solide ou encore avantageusement une portion obtenue après séparation plus ou moins importante de liquide.

Selon le prétraitement utilisé, le marc est sous forme solide avec plus ou moins d'humidité mais sans phase liquide ou bien il contient des phases solide et liquide, et dans ce dernier cas, la phase liquide peut être séparée en totalité ou en partie.

En fonction de la méthode de prétraitement utilisée, la partie solide représente entre 20 et 70% du poids du marc prétraité. Le marc utilisé pour la production d'enzymes présente un taux de matière sèche de 20-70%, et de façon très préférée de 40%-60% (ce qui correspond souvent à l'obtention d'un marc sous forme solide à l'issue du prétraitement).

Ladite partie solide est constituée de lignine, de composés minéraux, de cellulose et d'hémicellulose résiduelle non hydrolysée. La part de cellulose dans ladite partie solide est de 30 à 60% poids. La part de lignine dans ladite partie solide est de 20 à 50% poids. La part des composés minéraux et d'hémicellulose dans ladite partie solide est de 1 à 10% poids.

La part liquide dudit marc prétraité contient du xylose, des xyloligosaccharides, du mannose, de l'arabinose dans des proportions comprises entre 30 et 80%.

Dans un mode de réalisation préféré, le marc est utilisé directement, c'est-à-dire sans subir de traitement chimique ou biochimique. Ainsi l'invention n'utilise pas un marc prétraité ayant également subi une hydrolyse enzymatique. Un ou des traitements physiques sont possibles (séparation de liquide, chauffage, moyen de concentration...).

Dans un mode préféré, le marc est non lavé. Il peut dans certains cas être lavé pour détoxifier, de préférence en utilisant une quantité d'eau minimale.

Ladite phase b) de production est réalisée sur une durée comprise entre 70 et 200 h, de préférence entre 100 et 150 h.

De préférence, ladite phase b) de production opère à un pH compris entre 4 et 5 et à une température de 25 à 27°C.

À l'issue de l'étape de prétraitement, le marc prétraité est utilisé directement ou non dans la phase b) de production du cocktail enzymatique selon l'invention comme substrat carboné inducteur.

La phase b) de production est réalisée soit en mode continu par un ajout continu du marc avec un débit compris entre 0.3 et 0.8 gramme de matière sèche par litre de milieu et par heure (de préférence de 0.4 à 0.6 g/L/h et le plus souvent 0.5 g/L/h), soit en mode fed-batch par un ajout séquentiel du marc toutes les f heures, f étant compris entre 0,5heures et 48 heures, la quantité de marc ajoutée étant comprise entre 0.3f et 0.8f gramme de matière sèche par litre de milieu.

Cela signifie que si, par exemple, l'ajout se fait toutes les 12h, la quantité de marc ajoutée sera comprise entre 3.6 (i.e. 0.3x12) et 9.6 (i.e.0.8x12) g de matière sèche par litre de milieu, de préférence entre 4.8 et 8.4 g de matière sèche par litre de milieu.

Selon un mode de conduite préféré du fed-batch, l'ajout de marc prétraité est effectué en fonction du signal du % molaire de CO2 dans les gaz de sortie et de la mesure de pH du milieu. Une stabilisation du % du CO2 à ± 0,02% (pour une vvm de 0.5min-1) couplée à une augmentation du pH de 0.05 unité ou une augmentation de la pO2 d'au moins 5%, enclenche l'ajout séquentiel de marc.

En effet, suite à un ajout, on constate une augmentation du %CO2 dans les gaz de sortie, cela correspond à la consommation des sucres solubles présents dans le marc prétraité (essentiellement xylose et glucose). Ensuite les enzymes attaquent la cellulose, ce qui va induire la production de cellulases et le %CO2 diminue.

L'ajout est réalisé lorsqu'on observe un arrêt de la décroissance du % CO2 dans les gaz de sortie et une augmentation du pH de plus de 0,05 unité.

Le signal de pO2 (concentration d'oxygène dissous à saturation) est généralement maintenu élevé (au-dessus de 30% de la concentration à saturation en oxygène dans le milieu liquide à pression atmosphérique).

Lors de la conduite de procédé, on veillera de façon avantageuse à ce que la viscosité apparente du milieu reste inférieure à 10 Pa.s pour un taux de cisaillement de 10 s⁻¹ de préférence inférieure à 1 Pa.s.

En effet, la viscosité affecte négativement le transfert d'oxygène. Il est alors nécessaire d'augmenter fortement la puissance dissipée et/ou le débit d'aération pour assurer le transfert, ce qui augmente fortement la dépense énergétique et peut rendre le procédé difficilement extrapolable à l'échelle industrielle.

Le marc prétraité utilisable dans le procédé selon l'invention présente les caractéristiques suivantes :
- dans le test d'hydrolyse enzymatique réalisé avec un échantillon dudit marc ramené à 15% de matière sèche (MS), avec 10 mg d'enzymes CELLIC^{®} CTEC2 (commercialisée par Novozymes) par gramme de MS, à 50°C et pH 4.8, au bout de 96h le rendement d'hydrolyse est supérieur à 80 %. Le rendement d'hydrolyse est le rapport de la masse de sucres simples (tels que glucose, xylose) libérés par l'hydrolyse enzymatique divisé par la masse théorique maximale qui serait obtenue si toute la cellulose, les hémicelluloses et les oligomères issus du prétraitement étaient hydrolysés,
- dans le test de viscosité, est mis en suspension à 10% de MS, le marc prétraité présente une viscosité apparente inférieure à 1Pa.s, et de préférence inférieure à 0.15 Pa.s, pour un taux de cisaillement de 10 s-1. La mesure est réalisée avec un rhéomètre AR2000 de TA Instrument avec une géométrie de type ruban hélicoïdale comme décrit dans l'article « Experimental guidelines to optimize two crucial steps of lignocellulosic bioethanol production: a rheological approach » de Hénault et al. 2014.

La nature de la biomasse à l'origine du marc prétraité a une influence sur la production d'enzymes, et également sur les performances en hydrolyse enzymatique du procédé biochimique de traitement de la biomasse ligno-cellulosique. Les exemples montrent que le miscanthus est plus réactif que la paille, cette dernière produisant néanmoins des performances de bon niveau dans le procédé selon l'invention.

### Exemples

Les exemples démontrent que le mode de conduite et le type de marc utilisé ont une influence sur les performances du procédé.

### Exemple 1 : Utilisation du marc prétraité en mode batch : tout le marc est ajouté en début d'expérience.

La préculture du champignon est effectuée en fermenteur agité mécaniquement. Le milieu minéral a la composition suivante : KOH 1,66 g./L, H3PO4 85 % 2 mL/L, (NH4)2SO4 2,8 g/L, MgSO4, 7 H2O 0,6 g/L, CaCI2 0,6 g/L, MnSO4 3,2 mg/L, ZnSO4, 7 H2O 2,8 mg/L, CoCl2 10 4,0 mg/L, FeSO4, 7 H2O 10 mg/L, Corn Steep 1,2 g/L, anti-mousse 0,5 mL/L et addition de phtalate de potassium à 5 g.L-1 pour tamponner le pH.

Le fermenteur contenant le milieu minéral est stérilisé à 120 °C pendant 20 minutes.

Le fermenteur est ensemencé avec la souche de *Trichoderma reesei* CL847.

La croissance du champignon en préculture est faite en utilisant le glucose comme substrat carboné, à la concentration de 30 g.L-1. La croissance de l'inoculum dure de 2 à 3 jours et est effectuée à 28 °C dans un incubateur agité.

Le transfert vers le fermenteur de production de cellulase est réalisé lorsque la concentration résiduelle en glucose est inférieure à 15 g/L.

4 expériences ont été réalisées pour produire des enzymes en utilisant du miscanthus prétraité par explosion à la vapeur en condition acide:
- 2 expériences réalisées à 10 % de MS
- 2 expériences réalisées à 20% de MS

La production de cellulases est effectuée en fermenteur agité mécaniquement. Le milieu minéral a la composition suivante : KOH 1,66 g./L, H3PO4 85 % 2 mL/L, (NH4)2SO4 2,8 g/L, MgSO4, 7 H2O 0,6 g/L, CaCI2 0,6 g/L, MnSO4 3,2 mg/L, ZnSO4, 7 H2O 2,8 mg/L, CoCl2 10 4,0 mg/L, FeSO4, 7 H2O 10 mg/L, Corn Steep 1,2 g/L, anti-mousse 0,5 mL/L.

Le fermenteur contenant le milieu minéral est stérilisé à 120 °C pendant 20 minutes.

Le fermenteur est ensemencé à 10% (v/v) avec une préculture liquide de la souche de *Trichoderma reesei* CL847. Le pH est régulé à 5,5

Les expériences réalisées à 20% de MS ont échoué : il n'y a eu aucune production de cellulases. Le milieu était trop visqueux et/ou contenait trop d'inhibiteurs.

Les expériences réalisées à 10% de MS ont permis de produire 15 g/L de protéines au bout de 150 h soit une productivité de 0.1 g/L/h.

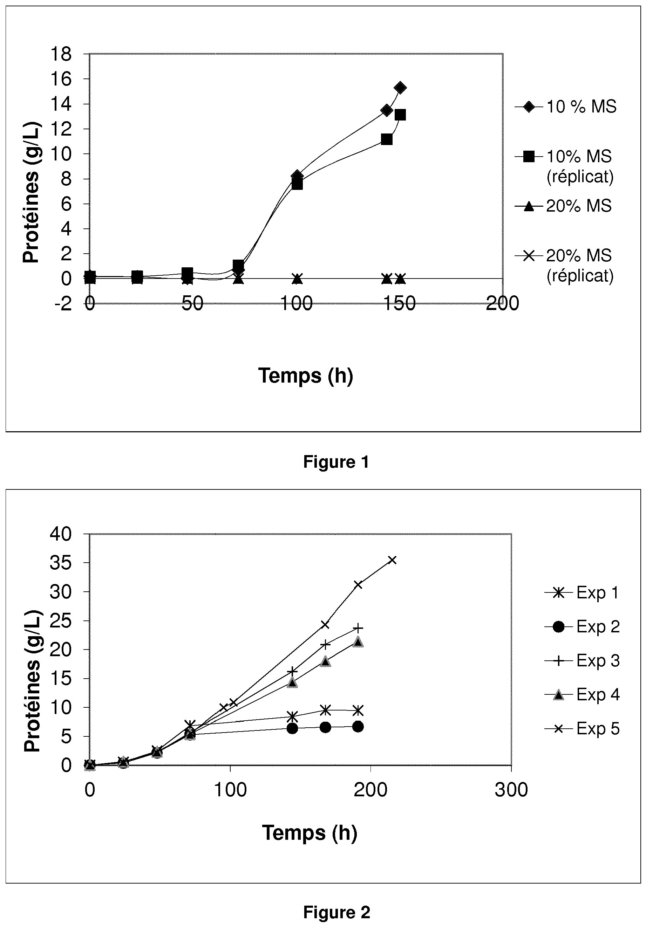

L'évolution de la concentration en protéines en mode batch avec de la biomasse lignocellulosique prétraitée en condition acide est illustrée figure 1.

### Exemple 2 : Expériences en mode fed-batch

5 expériences ont été réalisées (Exp 1 à 5).

La préculture du champignon *Trichoderma reesei* CL847 est effectuée comme dans l'exemple 1 mais avec 15 g/L de glucose comme unique substrat carboné. La production de cellulases débute après 24h en ajoutant le marc prétraité en mode fed batch c'est-à-dire par ajout
- de 6 g de matière sèche de marc par litre de milieu toutes 12 heures (Exp 3, Exp 4, Exp 5),
- ou de 12 g de matière sèche par litre avec la même fréquence (Exp 1 et Exp 2).

Les 5 expériences ont été réalisées en utilisant 3 substrats lignocellulosiques différents prétraités par explosion à la vapeur en condition acide et obtenus tels que (pas de séparation de liquide):
- Miscanthus 1 répondant au test d'hydrolyse et et au test de viscosité (viscosité apparente à 10 s-1 égale à 0.09 Pa.s) (Exp 1 et Exp 5)
- Miscanthus 2 ne répondant pas au test d'hydrolyse (rendement <70%)(Exp 2 et Exp 3)
- Paille de blé 1 répondant au test d'hydrolyse et au test de de viscosité (viscosité apparente à 10 s-1 égale à 1.1 Pa.s) (Exp 4)

Les tests d'hydrolyse et de viscosité sont ceux décrits précédemment.

La figure 1 montre l'évolution de la concentration en protéines de différentes expériences réalisées avec lesdits marcs :

| | |
|---|---|
| Exp 1 : | Marc miscanthus 1-conduite avec une quantité de marc 2 fois supérieure à celle de la conduite optimale |
| Exp 2 : | Marc miscanthus 2 - conduite avec une quantité de marc 2 fois supérieure à celle de la conduite optimale |
| Exp 3 : | Marc miscanthus 2 -conduite dite optimale |
| Exp 4 : | Marc paille de blé 1 -conduite dite optimale |
| Exp 5 : | Marc miscanthus 1 -conduite dite optimale |

Le protocole de conduite dite optimale pour la production de cellulases, utilisé dans Exp 3,4 ou 5 est le suivant :
Après une phase de croissance de 24h en mode batch avec du glucose à 15 g/L, des ajouts de marc de miscanthus ont été faits toutes les 12 heures environ avec un ajout de 6 g de matière sèche pour un litre de milieu. Au bout de 120h, la fréquence des ajouts a été ajustée en fonction du signal de CO2.

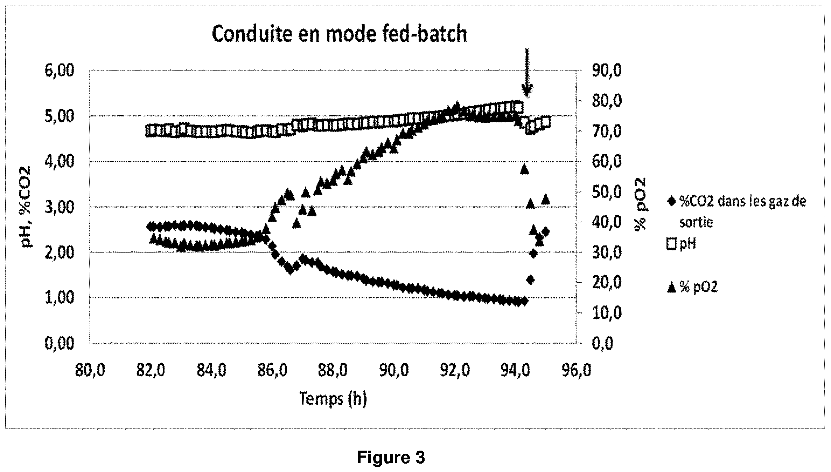

En effet, suite à un ajout, on constate une augmentation du %CO2 dans les gaz de sortie (figure 3), cela correspond à la consommation des sucres solubles présents dans le marc prétraité (essentiellement xylose et glucose). Ensuite les enzymes attaquent la cellulose qui va induire la production de cellulases (figure 2) et le %CO2 diminue.

L'ajout est réalisé lorsqu'on observe un arrêt de la décroissance du % CO2 dans les gaz de sortie et une augmentation du pH de plus de 0,05 unité.

Le signal de pO2 (concentration d'oxygène dissous à saturation) est maintenu élevé (au dessus de 30%) tout au long de l'expérience avec une faible puissance dissipée (inférieure à 1kW/m3).

Sur la figure 1, le marc est ajouté lorsque le pH remonte et que le CO2 arrête de baisser à raison de 6g de matière sèche par litre par ajout. La flèche correspond au moment où le marc est ajouté.

Les résultats sont tracés figure 2 et montrent l'importance à la fois du mode de conduite et du type de marc utilisé sur la performance du procédé.

En effet le procédé optimal (Exp 5 de la figure 2) permet d'aboutir à une concentration finale en cellulases de 35 g/L en 210 h, soit une productivité de 0.17 g/L/h.

Elle est donc supérieure de 70% à celle obtenue en batch présenté dans l'exemple 1.

Les autres expériences avec le même débit de fed-batch (conduite optimale) mais utilisant une paille prétraitée 1 (répondant aux tests) (Exp 4) ou du miscanthus 2 (ne répondant pas aux tests) (Exp3) ont abouti à une productivité inférieure respectivement de 32% et 25% à celle de l'expérience Exp 5.

Les expériences avec des débits de fed-batch deux fois supérieurs à l'optimal ont abouti à des mauvaises performances de production que ce soit avec le miscanthus prétraité 1 répondant aux tests (Exp1) ou avec le miscanthus prétraité 2 ne répondant pas aux tests (Exp 2).

Dans ces expériences, on a constaté que la pO2 est descendue en dessous de 10%. La paille prétraitée utilisée a une viscosité apparente à iso matière en suspension environ 12 fois supérieure à celle du miscanthus, ce qui peut expliquer la diminution de la pO2.

## Revendications

1. Procédé de production d'enzymes cellulolytiques ou hémicellulolytiques comprenant :
- une phase a) de croissance d'un microorganisme cellulolytique, ledit microorganisme cellulolytique étant un champignon de l'espèce *Trichoderma reesei,* en réacteur fermé, en présence d'au moins un substrat carboné de croissance à une concentration comprise entre 10 et 90 g/L, à une température de 25- 30°C et un pH de 4-5,5,
- une phase b) de production des enzymes dans laquelle au moins un substrat carboné inducteur est introduit à une température de 25-27°C et un pH de 4-5,
procédé dans lequel
- ledit substrat inducteur est un marc prétraité issu d'un procédé de prétraitement de matériau lignocellulosique, ledit prétraitement étant une explosion à la vapeur en condition acide, et ledit marc n'ayant pas subi d'hydrolyse enzymatique et étant introduit en mode fed-batch ou continu,
- ledit marc prétraité, mis en suspension à température ambiante, à 10% pds de MS, présente une viscosité apparente inférieure à 1 Pa.s pour un taux de cisaillement de 10 s⁻¹, de préférence inférieure à 0.15 Pa.s.,
- ledit marc est introduit à un débit compris entre 0,3 et 0,8 gramme de MS par litre de milieu et par heure en mode continu ; en mode fed-batch la quantité de marc ajoutée toutes les f heures, f étant compris entre 0,5h et 48h, est comprise entre 0.3f et 0.8f gramme de matière sèche par litre de milieu,
- ledit marc est formé d'un liquide et d'un solide, le solide contenant 20-70% de matière sèche, dont 20-50% de lignine, 30-60% pds de cellulose et 1-10% pds de composés minéraux et d'hémicellulose, et le liquide contenant 30-80% pds de sucres sous forme de xylose, de xyloligosaccarides, du mannose et de l'arabinose.

2. Procédé selon la revendication 1, dans lequel la viscosité apparente du milieu de l'étape b) reste inférieure à 10 Pa.s pour un taux de cisaillement de 10 s⁻¹, de préférence inférieure à 1 Pa.s.

3. Procédé selon l'une revendication 1 ou 2, dans lequel le marc a été lavé avant d'être introduit.

4. Procédé selon l'une revendication 1 ou 2, dans lequel le marc n'a pas été lavé avant d'être introduit.

5. Procédé selon l'une des revendications précédentes opérant en phase b) en l'absence de sucre ajouté.

6. Procédé selon l'une des revendications précédentes, dans lequel ledit marc prétraité est le seul substrat inducteur.

7. Procédé selon l'une des revendications précédentes, dans lequel dans la phase où le marc est ajouté, la concentration pO2 d'oxygène dissous à saturation en oxygène dans le milieu à pression atmosphérique est maintenue supérieure à 30%.

8. Procédé de traitement d'un marc prétraité issu d'un procédé de prétraitement de matériau lignocellulosique, dans lequel une partie du marc prétraité est utilisée en tant que substrat inducteur dans le procédé de production d'enzymes selon l'une des revendications précédentes, et dans lequel l'autre partie du marc prétraité est introduite dans une étape d'hydrolyse enzymatique en présence d'enzymes obtenues par le procédé de production d'enzymes selon l'une des revendications précédentes, l'hydrolysat obtenu étant envoyé dans une étape de fermentation éthanolique, l'effluent obtenu étant distillé pour séparer l'éthanol.

9. Procédé selon la revendication précédente, dans lequel ladite partie du marc prétraité est utilisée directement dans la phase b).

10. Procédé selon l'une des revendications 8 ou 9, dans lequel une partie ou la totalité du liquide contenu dans ladite partie du marc prétraité est séparée, et le marc obtenu est introduit dans la phase b).

## Patentansprüche

1. Verfahren zur Herstellung von Cellulose oder Hemicellulose abbauenden Enzymen, das Folgendes umfasst:
- eine Phase a) des Wachstums eines Cellulose abbauenden Mikroorganismus, wobei es sich beim Cellulose abbauenden Mikroorganismus um einen Pilz der Spezies *Trichoderma reesei* handelt, in einem geschlossenen Reaktor in Gegenwart mindestens eines kohlenstoffhaltigen Wachstumssubstrats mit einer Konzentration zwischen 10 und 90 g/l bei einer Temperatur von 25-30 °C und einem pH-Wert von 4-5,5,
- eine Phase b) der Herstellung von Enzymen, wobei mindestens ein induzierendes kohlenstoffhaltiges Substrat bei einer Temperatur von 25-27 °C und einem pH-Wert von 4-5 eingeführt wird,
wobei im Verfahren
- das induzierende Substrat ein vorbehandelter Trester aus einem Verfahren zur Vorbehandlung von lignocellulosehaltigem Material ist, wobei es sich bei der Vorbehandlung um eine Dampfexplosion unter sauren Bedingungen handelt und der Trester keiner enzymatischen Hydrolyse unterzogen worden ist und im Fed-Batch- oder im kontinuierlichen Betrieb eingeführt wird,
- der vorbehandelte Trester, der bei Umgebungstemperatur mit 10 Gew.-% TM suspendiert wird, bei einer Scherrate von 10 s⁻¹ eine Scheinviskosität von weniger als 1 Pa.s, vorzugsweise von weniger als 0,15 Pa.s, aufweist,
- der Trester mit einer Rate zwischen 0,3 und 0,8 g TM pro Liter des Mediums und pro Stunde im kontinuierlichen Betrieb eingeführt wird; im Fed-Batch-Betrieb die Menge des alle f Stunden zugegebenen Tresters, wobei f zwischen 0,5 h und 48 h beträgt, zwischen 0,3 f und 0,8 f Gramm Trockenmasse pro Liter des Mediums beträgt,
- der Trester aus einer Flüssigkeit und einem Feststoff besteht, wobei der Feststoff 20-70 % Trockenmasse enthält, wovon 20-50 % Lignin, 30-60 Gew.-% Cellulose und 1-10 Gew.-% mineralische Verbindungen und Hemicellulose sind, und die Flüssigkeit 30-80 Gew.-% Zucker in Form von Xylose, Xylooligosacchariden, Mannose und Arabinose enthält.

2. Verfahren nach Anspruch 1, wobei die Scheinviskosität des Mediums von Schritt b) bei einer Scherrate von 10 s⁻¹ unter 10 Pa.s, vorzugsweise unter 1 Pa.s, bleibt.

3. Verfahren nach einem der Ansprüche 1 oder 2, wobei der Trester vor seiner Einführung gewaschen wurde.

4. Verfahren nach einem der Ansprüche 1 oder 2, wobei der Trester vor seiner Einführung nicht gewaschen wurde.

5. Verfahren nach einem der vorhergehenden Ansprüche, wobei Phase b) ohne zugesetzten Zucker erfolgt.

6. Verfahren nach einem der vorhergehenden Ansprüche, wobei der vorbehandelte Trester das einzige induzierende Substrat ist.

7. Verfahren nach einem der vorhergehenden Ansprüche, wobei in der Phase, in welcher der Trester zugegeben wird, die Sättigungskonzentration pO2 des im Medium gelösten Sauerstoffs bei Atmosphärendruck über 30 % gehalten wird.

8. Verfahren zur Behandlung eines vorbehandelten Tresters aus einem Verfahren zur Vorbehandlung von lignocellulosehaltigem Material, wobei ein Teil des vorbehandelten Tresters als induzierendes Substrat im Verfahren zur Herstellung von Enzymen nach einem der vorhergehenden Ansprüche verwendet wird und wobei der andere Teil des vorbehandelten Tresters in einem Schritt einer enzymatischen Hydrolyse in Gegenwart von Enzymen eingeführt wird, die durch das Verfahren zur Herstellung von Enzymen nach einem der vorhergehenden Ansprüche erhalten werden, das erhaltene Hydrolysat einem Schritt der Ethanolfermentation zugeführt wird, der erhaltene Ablauf destilliert wird, um den Ethanol abzutrennen.

9. Verfahren nach dem vorhergehenden Anspruch, wobei der Teil des vorbehandelten Tresters direkt in Phase b) verwendet wird.

10. Verfahren nach einem der Ansprüche 8 oder 9, wobei ein Teil oder die Gesamtheit der Flüssigkeit, die im Teil des vorbehandelten Tresters vorhanden ist, abgetrennt wird und der erhaltene Trester in Phase b) eingeführt wird.

## Claims

1. Process for producing cellulolytic or hemicellulolytic enzymes comprising:
- a phase a) of growth of a cellulolytic microorganism, said cellulolytic microorganism being a fungus of the species *Trichoderma reesei,* in a closed reactor, in the presence of at least one carbon growth substrate at a concentration of between 10 and 90 g/L, at a temperature of 25-30°C and a pH of 4-5.5,
- a phase b) of production of enzymes, wherein at least one inducing carbon substrate is introduced at a temperature of 25-27°C and a pH of 4-5,
process, wherein
- said inducing substrate is a pretreated pomace originating from a process for the pretreatment of lignocellulosic material, said pretreatment being a steam explosion under acidic conditions, and said pomace not having been subjected to enzymatic hydrolysis and being introduced in fed-batch or continuous mode,
- said pretreated pomace, in a suspension at ambient temperature at 10% by weight DM, has an apparent viscosity of less than 1 Pa·s for a shear rate of 10 s⁻¹, preferably less than 0.15 Pa·s.,
- said pomace is introduced at a rate of between 0.3 and 0.8 grams DM per litre of medium and per hour in continuous mode; in fed-batch mode the amount of pomace added every f hours, f being between 0.5 h and 48 h, is between 0.3f and 0.8f grams of dry matter per litre of medium,
- said pomace is formed from a liquid and a solid, the solid containing 20-70% of dry matter, of which 20-50% is lignin, 30-60% by weight is cellulose and 1-10% by weight is mineral compounds and hemicellulose, and the liquid containing 30-80% by weight of sugars in the form of xylose, xyloligosaccarides, mannose and arabinose.

2. Process according to Claim 1, wherein the apparent viscosity of the medium in step b) remains less than 10 Pa·s for a shear rate of 10 s⁻¹, preferably less than 1 Pa·s.

3. Process according to one of Claims 1 and 2, wherein the pomace has been washed before being introduced.

4. Process according to one of Claims 1 and 2, wherein the pomace has not been washed before being introduced.

5. Process according to any of the preceding claims operating in phase b) in the absence of added sugar.

6. Process according to any of the preceding claims, wherein said pretreated pomace is the sole inducing substrate.

7. Process according to any of the preceding claims, wherein, in the phase in which the pomace is added, the pO2 concentration of dissolved oxygen at oxygen saturation in the medium at atmospheric pressure is maintained above 30%.

8. Process for treating a pretreated pomace originating from a process for the pretreatment of lignocellulosic material, wherein a part of the pretreated pomace is used as an inducing substrate in the process for producing enzymes according to any of the preceding claims, and wherein the other part of the pretreated pomace is introduced in a step of enzymatic hydrolysis in the presence of enzymes obtained by the process for producing enzymes according to any of the preceding claims, the hydrolysate obtained being supplied to a step of ethanolic fermentation, the effluent obtained being distilled to separate the ethanol.

9. Process according to the preceding claim, wherein said part of the pretreated pomace is used directly in phase b).

10. Process according to either one of Claims 8 or 9, wherein a part or all of the liquid contained in said part of the pretreated pomace is separated and the pomace obtained is introduced in phase b).
